# EUROPEAN PATENT APPLICATION

(11) **EP 2 351 521 A1**
(43) Date of publication of application: **03.08.2011**
(21) Application number: 10152351.2
(22) Date of filing: 02.02.2010
(51) Int. Cl.: A61B 5/11, G09F 3/02, A61B 19/00, A61G 7/057

(54) **Sensor system, sensor array and cover member**

(71) Applicant: Koninklijke Philips Electronics N.V., 5621 BA Eindhoven (NL)
(72) Inventor: Bruekers, Alphons, A.M.L., 5600 AE, Eindhoven (NL); Breebaart, Dirk, J, 5600 AE, Eindhoven (NL); Boughorbel, Sabri, 5600 AE, Eindhoven (NL)
(74) Representative: Damen, Daniel Martijn

(57) **Abstract**

A sensor system (100) is disclosed, comprising a sensor array (110), the sensor array (110) comprising a substrate layer (120, 150) and a plurality of individual sensor elements (130) for measuring a desired parameter arranged on said substrate layer (120, 150), the sensor elements (130) defining a sensor plane (140). The system comprises a control unit (170), a cover member (160) that is removably positionable over at least said substrate layer (120, 150), so as to cover said sensor plane (140), and a combination of at least one detector (200) and at least one data carrier (210, 211) that carries identification data relating to the cover member (210).

## Description

### FIELD OF THE INVENTION

The present invention relates to a sensor system comprising a cover member. The present invention further relates to a sensor array and a cover member.

### BACKGROUND OF THE INVENTION

Sensor systems are widely known and widely used in an almost unlimited number of applications and application areas. One particular type of sensor system comprises a so-called FLASC or Flexible Large-Area Sensor Carrier. Such a sensor system is for example known from Tekscan, which produces the clinical BPMS™ system (Body Pressure Measurement System). Typically such a system comprises flexible polyester sheets on which electrically conductive electrodes are positioned such that the electrodes face each other. The electrodes are arranged in a patterned manner. The known BPMS™ system is a pressure sensitive resistive system wherein between the conductive electrodes a coating is provided. When pressure is applied on the coating, its electrical resistance is changed and thus pressure data can be obtained. Different types of pressure sensitive sensor systems are also known, such as capacitive systems and systems using for example optical fibers and photo diodes.

An example of a pressure sensor system is shown in United States patent document US 2009/0070939 in which a system for the prevention of pressure ulcers with patients is disclosed. The system comprises a flat cushioning pad in which an array of pressure transducers is provided. The system further comprises a processor that is arranged to collect the signals that originate from the sensors in the array. The processor executes software that analyses the signals from the sensors and may provide the results to a (computer) display showing a graphical map of the pressure data. The system may be provided with a cover member that is positionable on top of the cushioning pad with the array of sensors and on which the patient lies during use of the system. The cover member may be a porous cloth attached over a layer of moisture-absorbing material. Preferably the material of the cloth is disposable and can be removed from the cushioning pad.

The inventors have realized that the presence of a cover member, for example the cushioning pad with the cloth of US 2009/0070939, on a sensor array may affect the operation of that sensor array and the data generated by it. In particular the inventors have realized that the physical properties of such a cover member will affect the operation of the sensor array. It can for example be easily understood, that a very thick or very rigid cover member on a sensor array will generate different data than a very thin or very flexible cover member on the same sensor array.

Similarly, in case the sensor array for example comprises one or more light sensors the level of transparency of the cover member will affect the amount of light that can be detected by the light sensors in the sensor array.

In the example disclosed in US 2009/0070939 the cushioning pad and the cloth are preferably made from disposable material. Hence, the pad and cloth may be replaced. Hence, it is a concern that by allowing the use of disposable and thus replaceable cover members it cannot be guaranteed that the next time the sensor system is used with a cover member, that the cover member has the same (or at least similar) properties. Hence, the results obtained from the sensor array may be differ at different occasions.

### OBJECT OF THE INVENTION

It would therefore be desirable to provide a sensor system of the above-mentioned type that can advantageously be used with different cover members. More in particular it would be desirable to provide a sensor system that is able to adapt to the specific cover member used in combination therewith.

### SUMMARY OF THE INVENTION

To better address one or more of these concerns, a sensor system is provided, that comprises a sensor array, the sensor array comprising a substrate layer and a plurality of individual sensor elements for measuring a desired parameter. The sensor array being arranged on said substrate layer, wherein the sensor elements define a sensor plane. The system further comprising a cover member that is removably positionable over at least said substrate layer, so as to cover said sensor plane. The sensor system further comprises a combination of at least one detector and at least one data carrier that carries identification data relating to the cover member. The detector is provided on one of the sensor array and the cover member, and the data carrier is provided on the other one of the sensor array and the cover member. The data carrier is detectable by the detector. The detector is further arranged to read said identification data.

By providing identification data that relates to the cover member information is available that can identify the cover member that is positioned on the sensor array. Such identification of the cover member can for example be done by means of a code that is contained in said identification data, whereby that code is compared with a comparison table present in the control unit. With the availability of the information concerning the cover member that is used, it is possible to take that information into account when assessing the sensor data. In the example of the system known from US 2009/0070939 the software used to interpret the sensor data may take the identification data into account when providing the eventual pressure data.

In an embodiment a control unit for controlling the sensor system is provided, wherein the control unit comprises a first input to receive sensor data from the sensor array, a second input to receive said identification data from the detector, a first output for providing a first output signal comprising, at least a part of, said identification data. In this manner both sensor data and identification data is available in a single control unit.

In an embodiment the control unit comprises a second output for providing a second output signal, which comprises, at least a part of, said sensor data. Hence, the control unit is able to provide control on the basis of both sensor data and identification data.

In an embodiment the data carrier carries identification data that relates to one or more physical properties of the cover member. In this manner certain physical properties such as thickness, rigidity, transparency to light, thermal conductivity etcetera can be directly provided upon reading the data carrier.

In an embodiment the system comprises a plurality of data carriers and/or one or more detectors. This provides a redundancy in the system, making it more robust.

In an embodiment the control unit is arranged to determine, on the basis of the identification data provided by the one or more detectors, a position of the cover member with respect to the sensor array. Hence, not only is information provided on the cover member and its properties, but also the manner in which it is used. This provides information on the relative position between the cover member and the sensor array, allowing for example the use of cover members with non-uniform properties.

In an embodiment said data carrier is chosen from a group comprising magnets, optical indicators, electrical contact elements and RFID components, and wherein said detector is chosen from a group comprising Hall elements, optical sensors, electrodes and RFID receivers. Said data carriers and corresponding detectors provide good detection results and good reading results, even when alignment between the data carrier and the respective detector is not optimal.

In an embodiment the cover member comprises a surface that faces away from the sensor array, which surface is provided with one or more visual and/or tactile indicators, which one or more visual and/or tactile indicators define an input area on said cover member, which input area corresponds to an operable area of the sensor array, and wherein said identification data includes data concerning the visual and/or tactile indicators. In this manner the properties that are identified by the identification data also includes data on areas on the surface of the cover, making the cover member useable as an input device. More in particular, the visual and/or tactile areas on the cover member correspond to an operable area of the sensor array, which makes it possible to use the sensor array as an input device as only certain parts of the sensor array are identified as operable.

In an embodiment the at least one detector is provided on the sensor array and the at least one data carrier is provided on the cover member. This provides a very practical embodiment.

In an embodiment the sensor array comprises one or more pressure sensitive elements and/or one or more temperature sensitive elements, and/or one or more light sensitive elements, and/or one or more moisture sensitive elements, and/or one or more sound sensitive elements of or any combination thereof. This provides a very versatile sensor system that can be used in a variety of applications.

In an embodiment the pressure sensitive elements are chosen from a group comprising: pressure sensitive resistive elements, pressure sensitive capacitive elements, piezo-electric elements, piëzo-resistive elements and optical elements, thereby providing a robust and cost-effective embodiment.

In an embodiment the sensor array is a Flexible Large-Area Sensor Carrier (FLASC), which provides a cost-effective substrate for carrying the sensor array.

In an embodiment the cover member is removably attachable to the sensor array, which ensures that during use the cover member stays attached to the sensor array.

In an embodiment, the cover member is provided with an accelerometer, so that it can be easily detected which side of the cover member is facing away from the sensor array and thus faces a user.

In a further aspect of the present invention a sensor array is provided that comprises a substrate layer and a plurality of individual sensor elements for measuring a desired parameter, which sensor elements are arranged on said substrate layer, the sensor array further comprising a detector that is arranged to detect identification data carried by a data carrier.

In a further aspect of the present invention a cover member is provided which comprises a data carrier, which data carrier is chosen from a group comprising magnets, optical indicators, electrical contact elements and RFID components.

### BRIEF DESCRIPTION OF THE DRAWINGS

Fig. 1 shows an exploded view of an exemplary embodiment of a sensor system;
Fig. 2 shows a schematic view of a pressure sensitive sensor array;
Fig. 3 shows a sensor array with a cover member, and
Fig. 4 shows an example of a data carrier with detector.

### DETAILED DESCRIPTION OF EXAMPLES

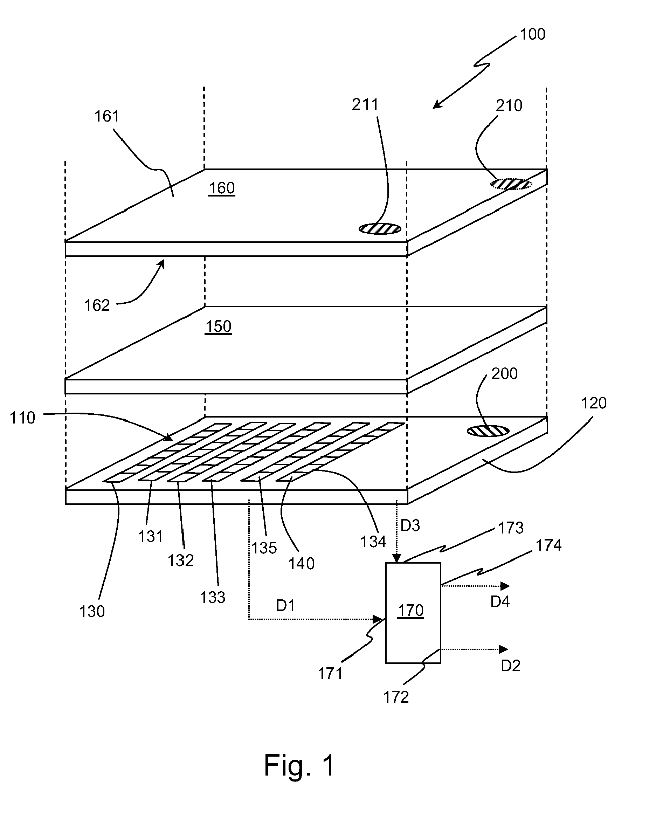

Figure 1 shows a schematic example of a sensor system 100 according to the invention. The sensor system 100 comprises a sensor array 110 which comprises or is provided on a substantially flat first substrate layer 120. The sensor array 110 comprises a plurality of individual sensor elements 130 which sensor elements 130 are arranged to measuring a desired parameter. As can be seen, the sensor elements 130 in the example shown define a substantially flat sensor plane 140. The sensor elements 130, in the example of Figure 1, are covered by a second substrate layer 150 of similar dimensions as the first substrate layer 120. The provision of the second substrate layer 150 is not essential, but may be convenient for protecting certain kinds of sensors, in particular electronic sensors.

The sensor elements 130 may all be sensors of a similar type. However, it is also possible that the sensor elements 130 comprise groups of different sensors depending on the desired parameter or parameters to be measured. The sensor elements 130 may for example comprise one or more pressure sensitive elements 131 and/or one or more temperature sensitive elements 132, and/or a plurality light sensitive elements 133, and/or a plurality moisture sensitive elements 134, and/or one or more sound sensitive elements 135, or any combination thereof. In the example of Figure 1 this is indicated as rows of sensor elements. However, it is to be understood that it is not essential that the sensors 130 are in a row-like manner, nor that sensors of a similar type are arranged in such a row-like manner. Instead, in case multiple sensors of different types are used, the distribution may be uniform or may be in the form of clusters of sensors where a certain parameter to be measures is expected to be present.

The sensor system 100 of Figure 1 further comprises a substantially flat cover member 160 that, in the example of Figure 1, can be placed on top of the second substrate layer 150. However, in case the second substrate layer 150 is not present the cover member 160 may be placed or arranged directly on the sensor array 110 and thus over at least the first substrate layer 120, so as to cover said sensor plane 140. The cover member 160 may be of any suitable material or type that allows the sensor elements 130 to measure the desired parameter(s).

A control unit 170 is further comprised in the sensor system 100 according to the invention. The control unit 170 comprises a first input 171 for receiving a first input signal D1 or sensor data generated by the sensor array 130. The control unit 170 may further comprise an output 174 for providing an output signal D4 comprising, at least part of, said sensor data D1. The output signal D4 may for example be supplied to a further control unit, a computer system or the like in which the output signal D4 is further manipulated to be suitable for display purposes on a display device for example.

Hereinafter the invention will be further explained by means of the example that the sensor array comprises pressure sensitive sensors only. As explained above, the present invention is not limited thereto.

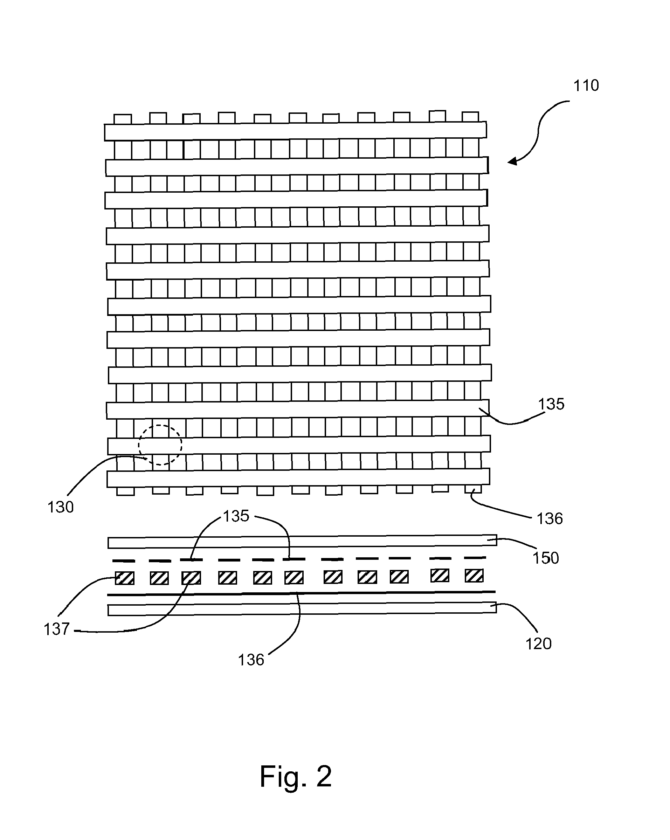

Figure 2 (upper half) shows a schematic example of a sensor array 110 in plan view, comprising a plurality of first electrodes 135 that are attached to the second substrate layer 150 and a plurality of second electrodes 160 that are attached to the first substrate layer 120. As can be seen in Figure 2, the first electrodes 135 and the second electrodes are orthogonal with respect to each other. However, other patterns are also conceivable and the present invention is not limited to the example shown in Figure 2. The first and second electrodes 135, 136 are electrically conductive electrodes. Between the first electrodes and the second electrodes at those locations where said electrodes cross, pressure sensitive resistive material 137 is provided. This is shown in the lower half of Figure 2, showing a schematic cross-sectional view. The pressure resistive material 137 prevents direct contact between the first and second electrodes 135, 136. The pressure resistive material 137 is for example a thin semi-conductive coating (ink), which material 137 has a varying electrical resistance when it is subjected to a varying pressure. The sensor array 110 hence comprises a plurality of pressure sensor elements 130 (indicated in Figure 2 with a dotted circle) that are formed at each intersection of a first electrode 135, a second electrode 136. The sensor array 110 is arranged to measure changes in current flow between the electrodes 135, 136 at each intersection when for example an object is placed on the second substrate layer 150. The measured changes in current flow (resulting from a change in the resistance of the pressure resistive material) represent the sensor data D1.

In the example of Figure 2, the sensor system utilizes pressure sensitive resistive material to form pressure sensitive sensor elements. Other solutions are also known, such as pressure sensitive capacitive sensor elements wherein the electrodes are separated by an air gap and the distance between the electrodes is a measure of the pressure exerted. Other solutions involve the use of piëzo-electric elements, or optical elements. Such alternative solutions are known per se and to the person skilled in the art. An example of a so-called FLASC or Flexible Large-Area Sensor Carrier as schematically shown in Figure 1 and 2 is the Tekscan BPMS^{TM} system. The BPMS^{TM} system may for example used for monitoring patients in bed to avoid the occurrence of ulcers.

When using a FLASC or sensor system 100 according to the invention, often the cover member 160 will be present. In the example of using the sensor system 100 to avoid ulcers with patients, the cover member may be a mattress or the like. Such a cover member 160 may affect the operation of the sensor array 110 and the data D 1 generated by it. In particular the physical properties of the cover member 160 will affect the operation of the sensor array 110. It can for example be easily understood, that a very thick or very rigid cover member will generated different data than a very thin or very flexible cover member on the same sensor array. Similarly, in case the sensor array 110 for example comprises one or more light sensors (as discussed above) the level of transparency of the cover member 160 will affect the amount of light that can be detected by the light sensors in the sensor array 110.

The sensor system 100 further comprises a combination of at least one detector 200 and at least one data carrier 210 that carries identification data relating to the type of cover member 160 that is used, i.e. is placed on the sensor array 110. More in particular, the data carrier 210 comprises information that identifies the cover member 160. In other words, the combination of the detector 200, that is able to detect the presence of the data carrier 210, and the data carrier 210 are arranged to provide identification data D3 to the control unit 170. This is indicated in Figure 1. In order to receive the identification data D3, the control unit 170 is provided with a second input 173. The identification data D3 may for example comprise data concerning the material(s) the cover member 160 is made of. It may also comprise data concerning its mechanical properties such as rigidity, thermal conductivity, transparency etcetera. It is also possible that the identification data D3 comprises a product code, that is provided to the control unit 170 which in turn comprises a comparison table in which table the provided identification data D3 is compared and the cover member 160 is identified.

When the identification data D3 is known to the control unit 170, the control unit 170 can take the type of cover member and/or its properties into account. This can for example be done by providing a second output signal D2 from a second output 172, wherein the second output signal comprises, at least part of, the identification data D3 or control data that has been established by the control unit 170 on the basis of and/or taking into account the identification data D3.

It is noted here that it is of course also possible that there is only one output and correspondingly only a single output signal that comprises both the sensor data and the identification data.

In the example shown in Figure 1, the data carrier 210 can only be properly detected when the data carrier 210 is in an area that is detectable by the detector 200. This means, that it is also possible with the embodiment as shown in Figure 1 to detect whether or not the cover member 160 is properly positioned on the sensor array 110. Such data can also be comprised in the identification data D3 and can for example be used to generate signal that indicates that the cover member 160 is positioned properly or not, for example by means of an audible or visible signal for a user of the sensor system 100.

In the example of Figure 1 the combination of one detector and one data carrier is shown. It is however also possible to use a combination of multiple data carriers and/or multiple detectors. In one such an example the cover member 160, which has an upper surface 161 and a lower surface 162 (see Figure 1), is provided with two data carriers 210, 211, whereby one data carrier 210 is located on the lower surface 162 of the cover member 160 and the other data carrier 211 is located on the upper surface 161 thereof. This is however not essential and the data carrier 211 may also be located in the material the cover member 160 is made of. When the respective data carriers 210, 211 additionally also carry data concerning their position on the cover member 160 (upper surface or lower surface), the system 100 can also detect what surface of the cover member 160 is facing upward. A practical application that the above combination of data carriers and detectors provides, is for example the use of the pressure sensitive sensor array 110 as a tool to control movement of toddlers for example. In such an application it is possible to define the upper surface 161 of the cover member 160 as a restricted area for example near a heat source such as an open furnace or fire place. When the cover member 160 is such a case is touched, it may trigger an alarm. Alternatively, the lower surface 162, which than becomes the upper surface when positioned upside down, may be defined as an area where the toddler must be confined to and an alarm is raised when the toddler leaves the cover member 160.

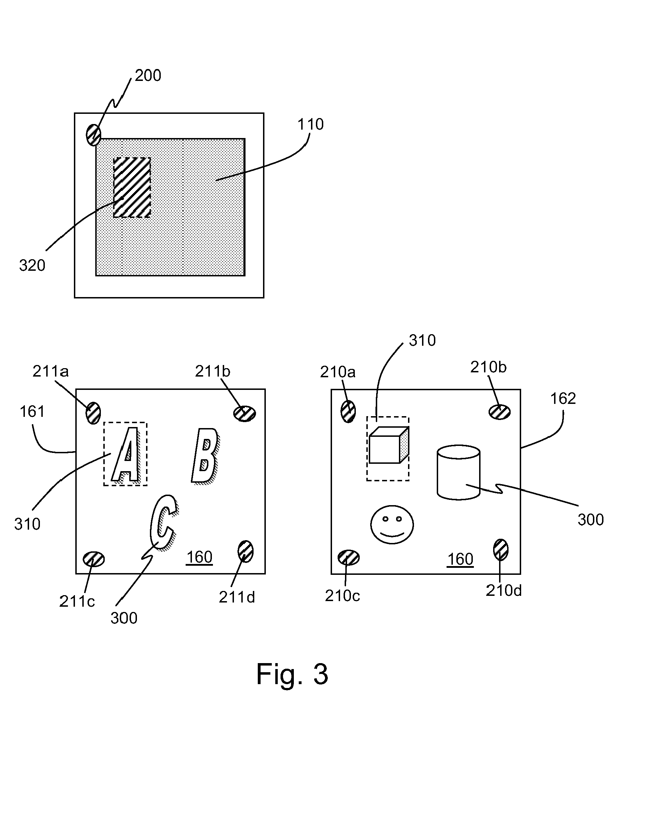

Another possibility that is provided when a plurality of data carriers and detectors are used, is to use the sensor system 100 as in input device, for example when used in combination with a personal computer or game console, that has even more applications than discussed above. A first example is shown with reference to Figure 3.

Figure 3 shows the substantially flat sensor array 110 or FLASC that is provided with the detector 200. Further an alternative cover member 160 is shown in two positions. In the left-hand part the cover member 160 has its first surface 161 facing away from the sensor array 110, in the right-hand part the cover member 160 has its second surface 162 facing away from the sensor array 110. The cover member 160 can, as was explained above, be positioned on the sensor array 110. This corresponds to what is shown in Figure 1. On the first surface 161 a first number of markings 300 are provided. These markings 300 comprise for example one or more letters of the alphabet (first surface 161) or general graphics (second surface 162). The markings 300 may be printed on the first surface 161, but can also be formed by sticking them onto the first surface 161 such that the markings 300 have a raised surface with respect to the first surface 161. In this manner visual and/or tactile indicators are provided.

As can be seen in Figure 3, the visual and/or tactile indicators or markings 300 define an input area 310 on said cover member 160, which input area 310 corresponds to an operable area 320 of the sensor array 110 when the cover member 160 is positioned on the sensor array 110. In order to properly function as an input device, wherein for example kids need to touch the correct letter of the alphabet on the cover member 160, the sensor system 100 must know which cover member is located on the sensor array 110 and what its orientation is with respect thereto. Similar as what has been described above, the detector 200 detects one of the four data carriers 211 a - 211 d that are provided on the first surface 161 of the cover member, or detects one of the four data carriers 210a - 210d that are provided on the second surface 162 of the cover member 160. All data carries 210a - 211d carry data identifying the cover member 160. Data carriers 210a - 210d identify that the second surface is facing up, whereas data carriers 211a - 211d identify that the first surface is facing up. The individual data carriers 210a - 211d each identify the rotational position of the cover member 160 with respect to the sensor array 110.

In this manner complete identification and detection of the mutual orientation of the cover member 160 and the sensor array 110 can be achieved. It shall be clear to a person skilled in the art, that the above described application of the invention is not limited to the example of a pressure sensitive sensor array. Similarly, a light sensitive, temperature sensitive and even sound sensitive sensor arrays can be used.

In this way the sensor system 100 may be used as an input device that has multiple applications, depending on the type of cover used and depending on its position on the sensor array 110.

In the examples shown, the data carrier(s) 210 is located on the cover member 160 and the detector(s) 200, which is an electronic element, is (are) located on the sensor array 110 or FLASC. This is the most practical embodiment as the data carrier 210 carries data that identifies the cover member 160. It is however also conceivable that the data carrier is located on the sensor array 110 and the combination of the data carrier 210 and the detector 200 provides information on the combination used, i.e. identifying the sensor array 110.

Examples of data carriers 210, 211 and detectors 200 that can be used are numerous. Some examples include the use of a series of small magnets as data carrier that are used to activate read-elements or so-called Hall elements on the sensor array. The pattern in which the small magnets are arranged on the cover member represent the identification data. Another example includes the use of a series of black and white dots or patterns provided on the cover member, the reflections of which are detected (read) by optical sensors provided on the sensor array. Yet a further example includes the use of electrical contacts on the cover member and electrodes on the sensor array which measure conductivity and a corresponding pattern is read that represents the identification data. Another example includes the use of so-called RFID elements and RFID detectors to provide and convey identification data.

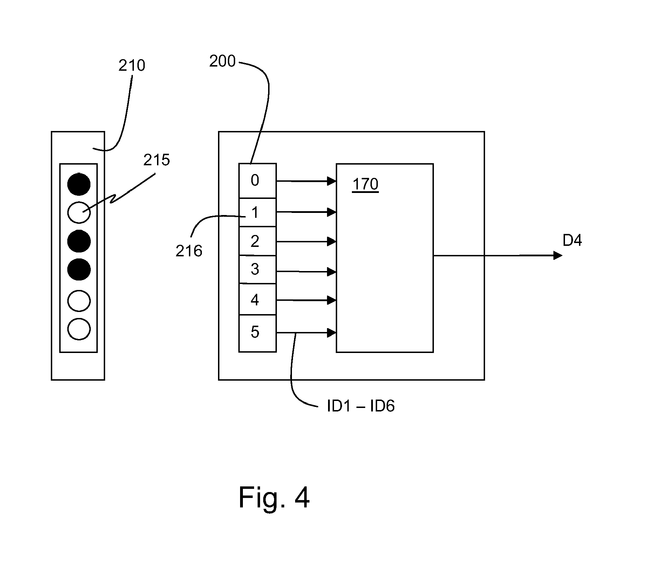

An example of a data carrier 210 and a detector or reader 200 is shown in Figure 4. Figure 4 shows an example of a data carrier 210 that comprises 6 individual data elements 215. In the example the data elements 215 are black and white dots representing for example a label that has 6 bits of information (010011). The detector or reader 200 has a corresponding number of individual read elements 216 numbered 0 to 5. It is to be understood, that the individual data elements 215 can also be of another type as indicated above. The data elements 215 are read by the read elements 216 represented by data ID1 - ID6, which data ID is supplied to the control unit 170. The control unit 170 in turn provides the output signal D4 identifying the cover member.

In the above the cover member and sensor array were shown to be of a generally rectangular shape. It is to be understood however that in principle any shape of the cover member and the sensor array are conceivable. Furthermore, both the cover member and the sensor array maybe provide with means to attach the cover member to the sensor array. Such means may include one or more of buttons, Velcro strips, ropes, zippers etcetera.

In the above examples the data carrier(s) is/are automatically read/detected. It is however to be understood that it is also possible that a user reads the data provided on the data carrier(s) and inputs said data in a control unit manually.

In the above it has been explained that the data carriers may also include data that can be used to identify which side of the cover member is facing upward, i.e. is facing away from the sensor array. Another solution to detect which surface of the cover member is facing away from the sensor array and thus forms an input area, is the use of an accelerometer. Such an accelerometer is arranged to indicate or to detect in which direction with respect to the accelerometer gravity is acting, and is thus able to identify the orientation of the object it is attached to with respect to gravity. The signal generated by the accelerometer can be supplied to a control unit as is explained above. This is in particularly convenient when the sensor system is used as an input device, as the coordinate system of the sensor array changes depending on which side thereof if facing the user.

Preferably, the data carrier and/or (data) detector may be detachable from the sensor array and/or the cover member. This allows washing/cleaning of the cover member and/or the sensor array for example.

In the above it has been disclosed that the cover member can be provided with an accelerometer. It is however also possible to provide the sensor array with an accelerometer also.

In the above the invention has been described by means of the example of a substantially flat cover member and a substantially flat sensor array. It is to be understood however that the invention is not limited to flat objects only. It is very well possible to extend the application of the invention to non-flat objects also. For example it is possible to provide a non-flat object that is provided with an array of sensors whereby the non-flat array of sensors can be covered by a cover member that, at least partly, envelops the non-flat sensor array. Furthermore, it is also possible that the cover member is in fact larger than the sensor array it is intended to (at least partly) cover. This means that the cover member may for example very well be draped around the edges of the sensor array.

It is also possible to provide the sensor array and/or the cover member with output means, such as LED's, loudspeakers, buzzers etcetera, which output means are for example controlled by the control unit of the sensor system.

While the subject-matter has been illustrated in the drawings and the foregoing description, such illustration and description are to be considered illustrative or exemplary and not restrictive; the subject-matter is not limited to the disclosed embodiments. Other variations to the disclosed embodiments can be understood and effected by those skilled in the art of practicing the claimed subject-matter, from a study of the drawings, the disclosure and the appended claims. Use of the verb "comprise" and its conjugations does not exclude the presence of other elements other than those stated in a claim or in the description. Use of the indefinite article "a" or "an" preceding an element or step does not exclude the presence of a plurality of such elements or steps. The Figures and description are to be regarded as illustrative only and do not limit the subject-matter. Any reference sign in the claims should not be construed as limiting the scope.

## Claims

1. A sensor system (100), comprising:
- a sensor array (110), the sensor array (110) comprising a substrate layer (120, 150) and a plurality of individual sensor elements (130) for measuring a desired parameter, which sensor elements (130) are arranged on said substrate layer (120, 150) and define a sensor plane (140), and
- a cover member (160) removably positionable over at least said substrate layer (120, 150), so as to cover said sensor plane (140),
**characterized in that**
the sensor system further comprises a combination of at least one detector (200) and at least one data carrier (210, 211) that carries identification data (D3) relating to the cover member (160),
wherein the detector (200) is provided on one of the sensor array (110) and the cover member (160), and the data carrier (210, 211) is provided on the other one of the sensor array (110) and the cover member (160),
wherein the data carrier (210, 211) is detectable by the detector (200),
wherein the detector (200) is further arranged to read said identification data (D3).

2. Sensor system (100) according to claim 1, further comprising a control unit (170) for controlling the sensor system (100), wherein the control unit (170) comprises a first input (171) to receive sensor data from the sensor array (110), a second input (173) to receive said identification data (D3) from the detector (200), a first output (172) for providing a first output signal (D2) comprising, at least a part of, said identification data (D3).

3. Sensor system (100) according to claim 2, wherein the control unit (170) further comprises a second output (174) for providing a second output signal (D4) comprising, at least a part of, said sensor data.

4. Sensor system (100) according to any of the previous claims, wherein the data carrier (210, 211) carries identification data that relates to one or more physical properties of the cover member (160).

5. Sensor system (100) according to any of the previous claims, wherein the system (100) comprises a plurality of data carriers (210a - 211d) and/or one or more of detectors (200).

6. Sensor system (100) according to claim 3, wherein the control unit (170) is arranged to determine, on the basis of the identification data provided by the one or more detectors (200), a position of the cover member (160) with respect to the sensor array (110).

7. Sensor system (100) according to any of the previous claims, wherein said data carrier (210, 211) is chosen from a group comprising magnets, optical indicators, electrical contact elements and RFID components, and wherein said detector (200) is chosen from a group comprising Hall elements, optical sensors, electrodes and RFID receivers.

8. Sensor system (100) according to any of the previous claims, wherein the cover member (160) comprises a surface (161, 162) that faces away from the sensor array (110), which surface (161, 162) is provided with one or more visual and/or tactile indicators (300), which one or more visual and/or tactile indicators (300) define an input area (310) on said cover member (160), which input area (310) corresponds to an operable area (320) of the sensor array (110), and wherein said identification data includes data concerning the visual and/or tactile indicators (300).

9. Sensor system (100) according to any of the previous claims, wherein the at least one detector (200) is provided on the sensor array (110) and the at least one data carrier (210, 211) is provided on the cover member (160).

10. Sensor system (100) according to any of the previous claims, wherein the sensor array (110) comprises one or more pressure sensitive elements (131) and/or one or more temperature sensitive elements (132), and/or one or more light sensitive elements (133), and/or one or more moisture sensitive elements (134), and/or one or more sound sensitive elements of or any combination thereof.

11. Sensor system (100) according to claim 10, wherein the pressure sensitive elements (131) are chosen from a group comprising: pressure sensitive resistive elements, pressure sensitive capacitive elements, piëzo-electric elements, piëzo-resistive elements and optical elements.

12. Sensor system (100) according to any of the previous claims, wherein the cover member (160) is removably attachable to the sensor array (110).

13. Sensor system (100) according to any of the previous claims, wherein the cover member (160) is provided with an accelerometer.

14. A sensor array (110), comprising a substrate layer (120, 150) and a plurality of individual sensor elements (130) for measuring a desired parameter, which sensor elements (130) are arranged on said substrate layer (120, 150), the sensor array (110) further comprising a detector (200) that is arranged to detect identification data (D3) carried by a data carrier (210, 211).

15. A cover member (160) comprising a data carrier (210, 211), which data carrier (210, 211) is chosen from a group comprising magnets, optical indicators, electrical contact elements and RFID components.
